**Europäisches Patentamt**

**European Patent Office**

**Office européen des brevets**

(19)

(11) **EP 1 262 559 A1**

(12) **EUROPEAN PATENT APPLICATION**

(43) Date of publication:
**04.12.2002 Bulletin 2002/49**

(51) Int Cl.⁷: **C12Q 1/26**, C12Q 1/28,
C12Q 1/32, A61B 10/00,
G01N 33/52

(21) Application number: **01113396.4**

(22) Date of filing: **01.06.2001**

(84) Designated Contracting States:
**AT BE CH CY DE DK ES FI FR GB GR IE IT LI LU
MC NL PT SE TR**
Designated Extension States:
**AL LT LV MK RO SI**

(71) Applicant: **SIRS-Lab GmbH
07745 Jena (DE)**

(72) Inventors:
• **Deigner, Hans-Peter, Dr.
69514 Laudenbach (DE)**

• **Russwurm, Stefan, Dr.
07743 Jena (DE)**
• **Friedrichs, Arnd, Dr.
07743 Jena (DE)**

(74) Representative: **Störle, Christian, Dr. et al
Geyer, Fehners & Partner,
Perhamerstrasse 31
80687 München (DE)**

(54) **Dermal patch for detecting lactate**

(57) A dermal patch (1) for detecting lactate is described wherein the patch comprises (a) means for adhering (2) the patch to the skin (3), and (b) one or more reagent layer(s) (4) containing the reagent(s) for determining lactate. Furthermore, a method for detecting lactate as well as the use of this dermal patch are described.

Fig. 1

EP 1 262 559 A1

**Description**

The invention relates to a dermal patch for detecting lactate, a method for detecting lactate and the use of the patch for detecting lactate in body liquids.

It is well-known that lactate is associated with various diseases and, furthermore, it is a reliable marker of anaerobic metabolism.

Lactate is an important metabolite that needs to be monitored rapidly in people who are sick enough to be in critical care units in hospitals, mainly to prevent heart attacks. Patients with cystic fibrosis have increased concentrations of lactate in their saliva. Lactate monitors are also used in diabetes control, control of exercise in rehabilitation and in food analysis.

Significant deviations of physical lactate levels can be seen after drug treatment. Therefore, lactate determination is also useful for drug therapy monitoring.

The lactate concentration is of particular importance in the supervision of the training of athletes, such as triathletes and marathon runners. Knowing one's lactate concentration is of importance not only in the supervision of the training of professional athletes, but also of people indulging in sports as a hobby, or people supposed to re-establish their physical performance by means of rehabilitational measures after diseases, said measures also including sports at a moderate level of endurance. Depending on the purpose served by such training, much of the training in endurance sports is to take place in the so-called aerobic range, i.e. a range wherein the oxygen taken in by respiration is sufficient to provide the required energy. If this is not the case, the training takes place in the so-called anaerobic range, wherein the amount of oxygen taken in is no longer sufficient to provide the required energy. A metabolite of the anaerobic provision of energy is lactate. Depending on the training purpose, training condition etc. it may also be desirable, however, to purposefully perform certain training units in the anaerobic range, but nevertheless most of the training should be carried out in the anaerobic range. Thus, the determination of lactate is important for the control of the training.

Several methods are known for determining lactate, which are essentially based on detecting lactate in the blood. The lactate determination in performance diagnostics of athletes is therefore either up to professional athletes or has to be carried out in laboratories being especially equipped for this purpose.

US 4,166,763 describes a method and an analytical element for detecting lactate which is oxidized by lactate oxidase to pyruvate and hydrogen peroxide. The quantity of lactate is determined by detecting the amount of hydrogen peroxide produced. The hydrogen peroxide in turn is detected colorimetrically using a peroxidase-catalyzed detection system. This method is carried out with a multilayer analytical element comprising (1) a spreading layer; (2) a reagent layer that is in fluid contact with the spreading layer under the condition of use and (3) optionally a support. The analytical element is adapted for use in an analytical system employing reflection techniques of spectrometric analysis. This prior art is focused on the detection of lactate in serum which is derived from whole blood.

However, the detection of lactate in serum and/or whole blood requires blood samples to be taken, which is unpleasant, on the one hand, and is often carried out by medically trained personnel, on the other hand. Furthermore, the detection of lactate according to US 4,166,763 is technically complicated, since it requires a spectrometrical analysis, which is carried out using special, complicated and expensive equipment.

Thus, the technical problem underlying the present invention is to provide an analytical element for detecting lactate and a method for detecting lactate which do not show the disadvantages of the analytical element and method according to the prior art.

According to the present invention, this is achieved by a dermal patch for detecting lactate, wherein the patch comprises (a) means for adhering the patch to the skin, and (b) one or more reagent layer(s) containing the reagent (s) for determining lactate.

Although for the sake of simplicity the terms lactate and pyruvate, respectively, are used hereinafter, it is to be understood that these terms also comprise lactic acid and pyruvic acid, respectively.

The present invention is directed to a dermal patch which can be directly attached to the skin of a human or animal whose lactate level is to be investigated. This attachment is achieved by the means for adhering the patch to the skin. The present invention is based on the finding that there is a correlation between the lactate concentration in sweat, saliva and, furthermore, other body liquids, on the one hand, to the concentration of lactate in blood on the other hand.

The dermal patch according to the present invention has one or more reagent layer(s) containing the reagent (s) for determining lactate. Such a reagent layer is, for example, described in the afore-mentioned prior art US 4,166,763 which is incorporated herein by reference.

According to a preferred embodiment of the present invention, the reagent(s) of the reagent layer comprise (s) an enzyme and optionally further educts for converting lactate to pyruvate and a further product as well as (a) chemical(s) for directly detecting the pyruvate or for detecting the further product. An example of such a further product is hydrogen peroxide.

As stated above, the reagent layer can contain enzymes, which may be immobilized on it. The reagent layer can be made of a gel material or of a membrane material. The reagent layer can contain, as further educts, reagents and chemicals required for the enzymatic conversion of lactate to pyruvate.

Chemical reactions which may be used in the present invention for detecting lactate are described hereinafter.

For example, lactate oxidase can be used in the patch according to the present invention to convert lactate to yield pyruvate and hydrogen peroxide according to the following equation:

$$lactate + O_2 + H_2O \rightarrow pyruvate + H_2O_2 \qquad \text{(Reaction I)}.$$

According to this equation, lactate reacts in the presence of lactate oxidase and oxygen to produce pyruvate arid hydrogen peroxide. The hydrogen peroxide can then be detected to determine the amount of lactate present in the sample of body liquid to be investigated.

Methods for detecting and/or quantifying hydrogen peroxide in assays, such as used in the present invention generally use a composition containing a substance having peroxidative activity, e.g., peroxidase and peroxidase-like substances, and material which undergoes a detectable change (generally a visible change) in the presence of hydrogen peroxide and the peroxidative substance.

A peroxidase is an enzyme which catalyzes a reaction wherein hydrogen peroxide oxidizes another substance. The peroxidases are generally conjugated proteins containing iron porphyrin. An example of a peroxidase is horseradish peroxidase. Also, certain synthetic peroxidases are available which can be used in the present invention.

For example, the hydrogen peroxide can be detected according to the following equation:

$$H_2O_2 + 4\text{-aminophenazon} + 4\text{-chlorphenol} \rightarrow chinonimine\ dye \qquad \text{(Reaction II)}.$$

This reaction is catalyzed by a peroxidase.

The enzymatic activity is greatest between pH 7-9.7 and, therefore, having and maintaining such a pH value in the dermal patch is beneficial.

The extent of the reaction, i.e. the formation of the dye, is directly proportional to the lactate concentration. The amount of lactate is determined by a color change and color intensity which is directly proportional to the quantity of lactate present in the body liquid to be investigated.

Alternatively, lactate can be transformed to pyruvate according to the following equation:

$$lactate + NAD \rightarrow pyruvate + NADH_2 \qquad \text{(Reaction III)}.$$

This reaction is catalyzed by lactate dehydrogenase. A possible reaction mixture for converting lactate to pyruvate by means of lactate dehydrogenase may comprise the following: Tris-buffer pH 9.5: 110 mmol/l; lactate dehydrogenase: 18 U/ml and NAD: 2.8 mmol/l. The pyruvate can be further processed. For example, reaction with pyruvate oxidase can yield a peroxide which can be detected as described above. Also in this reaction sequence, the degree of developed color is proportional to the amount of pyruvate present.

Furthermore, the pyruvate which may be produced according to the afore-outlined reactions I and III, or any other reaction, can be determined by reacting it directly with a hydrazin compound producing a visible color, for example, 2,4-dinitrophenylhydrazine. After adding dilute alkali, for example (1) N-alkali, the visible color produced is proportional to the quantity of pyruvate.

With the dermal patch according to the present invention, the color produced can be measured by eye and quantitated with reference to a standard color chart. It is favourable to provide said standard color chart directly on the upper surface of the dermal patch so as to enable a direct comparison of the color thus generated with the colors of the color chart. Thus, in a particularly advantageous manner, a very easy evaluation of the analytical result is possible.

The detection of lactate may also be effected by means of common antibody reactions, wherein antibodies specific for pyruvate and/or for the further products generated by the enzymatic conversion can be used in a common way.

In a preferred embodiment of the present invention, the means for adhering the dermal patch to the skin comprise an adhesive and/or an adhesive tape. The use of such means for adhering in band aids is well-known. In a particularly advantageous manner, they are especially well-attachable, while also being easily removable after use.

In another preferred embodiment of the dermal patch, the reagent layers comprise a first reagent layer and a second reagent layer. In particular, the first reagent layer can contain the enzyme and optionally the further educts for converting lactate to pyruvate and the further products. The second reagent layer contains the chemicals for directly detecting pyruvate or for detecting the further products. The terms "first reagent layer" and "second reagent layer" are to be understood such that the first reagent layer is situated more closely to the skin than the second reagent layer. This separation of the reagent layers into first and second reagent layers allows a spatial separation, similar to the spatial separation in different test tubes, of the reactions taking place in the detection of lactate. Thus, disturbing interactions can be avoided during the individual reactions, advantageously resulting in increased accuracy of measurement and reliability.

According to a further preferred embodiment of the present invention, a membrane which is permeable for the further products is positioned between the first and the second reagent layer in order to achieve the spatial separation. If the further product is hydrogen peroxide, the membrane separating the first reagent layer from the second reagent layer, is permeable for hydrogen peroxide, but not for lactate and/or other substances with a molecular weight of 180 Dalton or more. This permeable membrane between the first reagent layer and the second reagent layer can be made, for example, of cellulose acetate. Thus, the above-described separation is achieved in a particularly favourable manner, while at the same time ensuring that the compounds required for carrying out said quantitative determination migrate from the first reaction layer to the second reaction layer.

According to another preferred embodiment of the present invention, the dermal patch comprises a support, which can be made, for instance, of polycarbonate. For example, the support can be provided between the means for adhering the patch and the reagent layer, or the first reagent layer. Such a support imparts more form stability to the dermal patch as a whole, while still maintaining sufficient flexibility for the patch to be well-adjustable to the surface of the skin. In this manner, particularly good handling of the dermal patch is achieved.

However, the term "between" is not to be understood such that the support is necessarily in direct contact with the means for adhering the patch to the skin and the reagent layers. Rather, the term "between" means that still further membranes, e.g. hydrophilic, permeable membranes, may be disposed between the means for adhering the patch to the skin and the support and/or between the support and the reagent layers.

According to a further preferred embodiment of the present invention, the dermal patch, in particular the reagent layer, has a defined volume. Based on the knowledge of the defined volume, a particularly exact quantitative determination of the lactate concentration in the body liquid to be examined is possible.

For example, a sponge and/or filter paper may be provided between two liquid-permeable membranes, which may, e.g., be prepared from polycarbonate, so as to achieve a defined volume. This configuration of a sponge and/or filter paper between membranes allows the accumulation of liquids in this configuration up to a defined volume. Based on a liquid stream starting from the skin layer to the surface of the dermal patch disposed at the maximum distance from the skin layer, it is favourable to provide a sponge and/or filter paper assembly between the membranes before the reagent layer or reagent layers in the flow direction, such that the volume is precisely determined before carrying out the reaction.

The dermal patch according to the present invention may be prepared in a conventional manner, e.g. by immobilizing enzymes and impregnating the individual components of the patch with liquids which contain chemicals required for carrying out said reactions. The individual layers of the patch may be joined together by laminating and/or glueing.

The dermal patch according to the present invention has sufficient permeability so as to permit body liquids, containing basically water, lactate and optionally other constituents, to escape through the patch, in particular through the support layer to the reaction layer.

It has further turned out to be beneficial to apply a membrane that is not permeable to water and dyes as the uppermost layer of the dermal patch, furthest removed from the skin. This advantageously ensures that soiling, e.g. of clothes, is avoided. Further, it is also favourable to use a transparent membrane as the uppermost layer, in order to ensure particularly good observation of the color reaction.

A dermal patch according to the present invention can comprise the following layers (wherein layer i) is next to the skin): i) a polycarbonate membrane, with means for removably securing the patch in fluid communication with the skin; ii) a sponge or filter paper; iii) a polycarbonate membrane (optional); iv) (an) enzyme(s) immobilized in a gel or on a membrane, butter salts; v) a cellulose acetate membrane, permeable for hydrogen peroxide; vi) a layer containing reagents for dye forming reactions with hydrogen peroxide, immobilized peroxidase (optional), and vii) a transparent membrane, not permeable for dyes or reagents.

The dermal patch according to the present invention has a number of advantages. Thus, it enables a very easy determination of lactate in sweat or saliva, wherein an exact and reliable quantitative determination of lactate by comparing the color resulting from the color reaction with standard color charts is possible. Moreover, the patch according to the invention also allows a non-invasive determination of lactate, which means that no samples of body liquids, such as blood, need to be taken, which is generally unpleasant and cumbersome and often requires the presence of experienced personnel. Since the patch according to the present invention only needs to be attached to the skin, its use at home or directly while training is possible without requiring medical supervision. A reliable and simple detection of lactate requires only a small amount of body liquid, in particular sweat. The patch according to the invention is available at low cost, since it may be prepared from low-cost starting materials in a simple, quick and easy manner and in great numbers. The lactate measurement using the patch according to the invention may be carried out within few minutes, e.g. 1 to 20 minutes, in particular about 4 minutes.

The present invention further relates to a method for detecting lactate comprising adhering the dermal patch according to the present invention to the skin of a human or an animal and detecting the lactate with the reagent layer.

When carrying out the method according to the present invention, it is sufficient to attach the dermal patch according to the present invention to the skin, which contains the body liquid to be examined, in particular sweat. As reaction temperature for the detection reactions described in more detail above, body temperature is fully sufficient. As also described above, the reaction is complete already after a few minutes, in particular 1 to 20 minutes, especially about 4 minutes. The above-described detection reactions for determining lactate produce dyes, the quantity of the dyes produced and thus the color intensity being directly proportional to the amount of lactate present in the body liquid being analyzed. A comparison of the color thus generated with a standard color chart enables a precise determination of the lactate quantity.

The method according to the invention allows a rapid, low-cost and precise determination of the lactate concentration in body liquids, in particular sweat.

Further, the present invention also relates to the use of the patch according to the present invention for detecting lactate in body liquids, in particular sweat and saliva. Said use of the patch according to the invention is principally possible whenever lactate needs to be determined. The dermal patch according to the present invention may be employed, above all, for performance diagnostics in sports, in particular in professional sports, but also in sports merely performed as a hobby.

The present invention shall be described now with reference to the drawing, which serves to illustrate the present invention without restricting it and which shows an embodiment of the dermal patch according to the present invention.

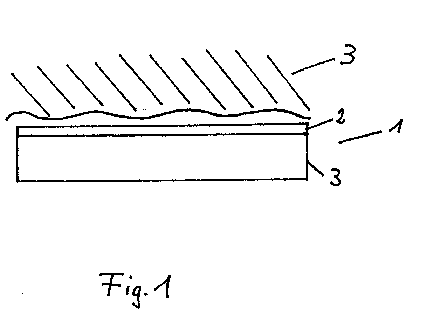

Fig. 1 shows an embodiment of the dermal patch 1 according to the present invention, comprising means for adhering 2 the patch 1 to the skin 3 and a reagent layer 4 containing the reagents for determining lactate.

**Claims**

1. A dermal patch (1) for detecting lactate, the patch comprising

    (a) means for adhering (2) the patch to the skin (3), and
    (b) one or more reagent layer(s) (4) containing the reagent(s) for determing lactate.

2. The patch according to claim 1, wherein the reagent(s) comprise an enzyme and optionally further educts for converting lactate to pyruvate and a further product as well as (a) chemical(s) for directly detecting the pyruvate or for detecting the further products.

3. The patch according to claim 2 wherein the further product is hydrogen peroxide.

4. The patch according to anyone of claims 1 to 3, wherein the means for adhering (2) comprise an adhesive and/or an adhesive tape.

5. The patch according to anyone of claims 1 to 4 wherein the reagent layers (4) comprise a first reagent layer and a second reagent layer.

6. The patch according to claim 4, wherein a membrane, which is permeable for the further products, is positioned between the first and the second reagent layer.

7. The patch according to claims 5 and 6, wherein the first reagent layer contains the enzyme and optionally the further educts for converting lactate to pyruvate and the further products and the second reagent layer contains the chemicals for directly detecting the pyruvate or for detecting the further products.

8. The patch according to anyone of the preceding claims, wherein a support layer is provided between the means for adhering (2) the patch and the reagent layer(s) (4).

9. The patch according to anyone of the preceding claims, wherein the reagent layer(s) (4) has (have) a defined volume.

**10.** A method for detecting lactate comprising adhering the patch according to anyone of claims 1 to 8 to the skin of a human or an animal and detecting the lactate by the reagent layer.

**11.** Use of the patch according to anyone of claims 1 to 8 for detecting lactate in body liquids.

**12.** Use according to claim 10, wherein the body liquid is sweat.

Fig. 1

**European Patent Office**

## EUROPEAN SEARCH REPORT

Application Number

EP 01 11 3396

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 99 13336 A (MITCHEN JOEL R ;ARONOWITZ JACK L (US); TECHNICAL CHEMICALS & PRODU) 18 March 1999 (1999-03-18)<br>* page 5, line 9 - page 6, line 14 *<br>* page 7, line 14 - line 20 *<br>* page 18, line 12 - line 13 *<br>* page 29, line 30 - page 30, line 14 *<br>* page 34, line 6 - line 22 *<br>* figure 1 * | 1,2,4, 10-12 | C12Q1/26<br>C12Q1/28<br>C12Q1/32<br>A61B10/00<br>G01N33/52 |
| X | US 5 817 011 A (SCHOENDORFER DONALD W) 6 October 1998 (1998-10-06)<br>* figures 1-11 *<br>* column 16, line 10 - line 67 *<br>* column 8, line 63 - column 9, line 8 *<br>* examples 4,5 * | 1,4,8-12 | |
| A | US 5 183 741 A (ARAI FUMINORI ET AL) 2 February 1993 (1993-02-02)<br>* abstract *<br>* column 2, line 50 - line 66 *<br>* column 5, line 30 - line 42 *<br>* column 7, line 53 - line 61 * | 1-3,5-7 | TECHNICAL FIELDS SEARCHED (Int.Cl.7)<br><br>C12Q<br>A61B<br>G01N |
| D,A | US 4 166 763 A (ESDERS THEODORE W ET AL) 4 September 1979 (1979-09-04)<br>* the whole document * | 1-12 | |

The present search report has been drawn up for all claims

| Place of search | Date of completion of the search | Examiner |
|---|---|---|
| THE HAGUE | 21 March 2002 | Tuynman, A |

EPO FORM 1503 03.82 (P04C01)

**ANNEX TO THE EUROPEAN SEARCH REPORT
ON EUROPEAN PATENT APPLICATION NO.** EP 01 11 3396

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2002

| Patent document cited in search report | | | Publication date | Patent family member(s) | | Publication date |
|---|---|---|---|---|---|---|
| WO 9913336 | A | | 18-03-1999 | AU | 9485098 A | 29-03-1999 |
| | | | | BR | 9815657 A | 20-11-2001 |
| | | | | CA | 2303129 A1 | 18-03-1999 |
| | | | | CN | 1301347 T | 27-06-2001 |
| | | | | EP | 1015887 A1 | 05-07-2000 |
| | | | | JP | 2001516056 T | 25-09-2001 |
| | | | | NO | 20001126 A | 27-04-2000 |
| | | | | WO | 9913336 A1 | 18-03-1999 |
| US 5817011 | A | | 06-10-1998 | US | 5441048 A | 15-08-1995 |
| | | | | US | 5203327 A | 20-04-1993 |
| | | | | US | 4957108 A | 18-09-1990 |
| | | | | US | 5899856 A | 04-05-1999 |
| | | | | AU | 677036 B2 | 10-04-1997 |
| | | | | AU | 5951194 A | 04-07-1994 |
| | | | | CA | 2151470 A1 | 23-06-1994 |
| | | | | EP | 0676051 A1 | 11-10-1995 |
| | | | | JP | 8504513 T | 14-05-1996 |
| | | | | US | 5438984 A | 08-08-1995 |
| | | | | US | 5944662 A | 31-08-1999 |
| | | | | WO | 9414062 A1 | 23-06-1994 |
| | | | | US | 5465713 A | 14-11-1995 |
| | | | | US | 5638815 A | 17-06-1997 |
| | | | | US | 5676144 A | 14-10-1997 |
| | | | | US | 5817012 A | 06-10-1998 |
| | | | | AT | 137583 T | 15-05-1996 |
| | | | | AU | 654823 B2 | 24-11-1994 |
| | | | | AU | 8313791 A | 17-03-1992 |
| | | | | CA | 2088921 A1 | 16-02-1992 |
| | | | | DE | 69119231 D1 | 05-06-1996 |
| | | | | DE | 69119231 T2 | 21-11-1996 |
| | | | | EP | 0543867 A1 | 02-06-1993 |
| | | | | JP | 3017286 B2 | 06-03-2000 |
| | | | | JP | 6503875 T | 28-04-1994 |
| | | | | WO | 9203731 A1 | 05-03-1992 |
| | | | | US | 5445147 A | 29-08-1995 |
| | | | | AT | 129868 T | 15-11-1995 |
| | | | | DE | 68924779 D1 | 14-12-1995 |
| | | | | DE | 68924779 T2 | 05-06-1996 |
| | | | | EP | 0433382 A1 | 26-06-1991 |
| | | | | JP | 3051419 B2 | 12-06-2000 |
| | | | | JP | 4500559 T | 30-01-1992 |
| | | | | WO | 9002511 A1 | 22-03-1990 |
| | | | | US | 5076273 A | 31-12-1991 |
| US 5183741 | A | | 02-02-1993 | JP | 1713505 C | 27-11-1992 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 01 11 3396

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2002

| Patent document cited in search report | | Publication date | Patent family member(s) | | Publication date |
|---|---|---|---|---|---|
| US 5183741 | A | | JP | 4000638 B | 08-01-1992 |
| | | | JP | 60082859 A | 11-05-1985 |
| | | | DE | 3479900 D1 | 02-11-1989 |
| | | | EP | 0137521 A2 | 17-04-1985 |
| US 4166763 | A | 04-09-1979 | BE | 861713 A1 | 09-06-1978 |
| | | | CA | 1095818 A1 | 17-02-1981 |
| | | | DE | 2755035 A1 | 15-06-1978 |
| | | | FR | 2392384 A1 | 22-12-1978 |
| | | | GB | 1560246 A | 30-01-1980 |
| | | | JP | 1305557 C | 28-02-1986 |
| | | | JP | 53105292 A | 13-09-1978 |
| | | | JP | 58004557 B | 26-01-1983 |
| | | | US | RE32016 E | 29-10-1985 |